(19) Europäisches Patentamt
European Patent Office
Office européen des brevets

(11) Veröffentlichungsnummer : **0 028 327**
**B1**

(12) **EUROPÄISCHE PATENTSCHRIFT**

(45) Veröffentlichungstag der Patentschrift : **03.11.82**

(21) Anmeldenummer : **80106200.1**

(22) Anmeldetag : **11.10.80**

(51) Int. Cl.³ : **C 07 C 51/60**, C 07 C 53/40, C 07 C 53/48, C 07 C 57/66, C 07 C 63/10

(54) **Verfahren zur Herstellung von Carbonsäurehalogeniden.**

(30) Priorität : **26.10.79 DE 2943433**

(43) Veröffentlichungstag der Anmeldung : **13.05.81 (Patentblatt 81/19)**

(45) Bekanntmachung des Hinweises auf die Patenterteilung : **03.11.82 Patentblatt 82/44**

(84) Benannte Vertragsstaaten : **BE CH DE FR GB IT LI**

(56) Entgegenhaltungen :
**AT B 72 292**
**DE C 638 306**
**DE C 687 670**
**FR A 2 232 532**
**FR A 2 254 547**

(73) Patentinhaber : **BAYER AG**
**Zentralbereich Patente, Marken und Lizenzen**
**D-5090 Leverkusen 1, Bayerwerk (DE)**

(72) Erfinder : **Rauchschwalbe, Günter, Dr.**
**Hofstrasse 28**
**D-5000 Köln 80 (DE)**
Erfinder : **Heydkamp, Wolfgang, Dr.**
**Grüner Weg 18**
**D-5090 Leverkusen 1 (DE)**
Erfinder : **Mannes, Karl, Dr.**
**Kurt-Schumacher-Ring 119**
**D-5090 Leverkusen (DE)**
Erfinder : **Mayer, Dietmar, Dr.**
**Hamberger Strasse 51**
**D-5090 Leverkusen (DE)**

**EP 0 028 327 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verfahren zur Herstellung von Carbonsäurehalogeniden

Die Erfindung betrifft ein Verfahren zur Herstellung von Carbonsäurehalogeniden aus den zugehörigen Carbonsäuren und einem Säurehalogenid in Anwesenheit von Aktivkohle.

Es ist bekannt, daß Carbonsäurechloride aus Carbonsäuren und Thionylchlorid hergestellt werden können (Houben-Weyl, Methoden der organischen Chemie, Band VIII, Seite 467-469, Georg Thieme Verlag Stuttgart, 1952). Einige Carbonsäuren, beispielsweise die Dichloressigsäure, reagieren jedoch mit Thionylchlorid nur langsam und/oder in schlechten Ausbeuten, andere beispielsweise Trichloressigsäure, reagieren unter den genannten Bedingungen praktisch nicht (J. Am. Chem. Soc. *50*, 145 (1928)).

Üblicherweise wird ein Überschuß von Thionylchlorid eingesetzt, beispielsweise 1,5 Mol Thionylchlorid pro Mol Carbonsäure (Organicum, 5. Auflage, Seite 408, VEB Deutscher Verlag der Wissenschaften 1965), dessen spätere Abtrennung von leicht flüchtigen Säurechloriden wie Acetylchlorid oder Chloracetylchlorid Schwierigkeiten bereitet und einen zusätzlichen Aufwand erfordert, so daß schlechte Ausbeuten an reinem Material erzielt werden, obwohl die Rohausbeute an Thionylchloridhaltigem Produkt gut sein kann (beispielsweise 55 % Chloracetylchlorid, J. Am. Chem. Soc. loc. cit.). Wird dagegen nur eine äquivalente Menge oder ein geringer molarer Überschuß an Thionylchlorid eingesetzt, so erhält man nur mäßige Ausbeuten, beispielsweise an Chloracetylchlorid oder Dichloracetylchlorid, die zudem ebenfalls unrein sein können. Es ist auch empfohlen worden, bei der Herstellung von Chloracetylchlorid das Thionylchlorid im Unterschuß einzusetzen (Chemical News, Vol. *122*, 220 (1921)), doch sind auch dann die Ausbeuten nur mäßig (etwa 80 %, bezogen auf eingesetzte Chloressigsäure).

Trichloressigsäure reagiert selbst mit überschüssigem Thionylchlorid nicht (J. Am. Chem. Soc., loc. cit. ; J. Chem. Soc. *1953*, 2 117). Weiterhin ist empfohlen worden, Thionylchlorid und Monochloressigsäure in der Gasphase umzusetzen und das entstehende Säurechlorid laufend aus der Reaktionszone zu entfernen (russische Patentanmeldung 144 475). Dieses Verfahren erfordert jedoch aufwendige technische Einrichtungen.

Umsetzungen von Carbonsäuren mit 1,5-4 Mol Phosgen pro Mol Säure in der Gasphase an Aktivkohlen werden weiterhin in den Patentanmeldungen FR 2 232 532 und FR 2 254 547 beschrieben. Hierin wird darauf verwiesen, daß in der Flüssigphase auch in Gegenwart von Katalysatoren nur unreine Produkte erhalten werden und im Falle der Chloressigsäure eine Reaktion selbst bei Temperaturen bis 150 °C nicht erzielt wird (genannte FR '532, Seite 1, Zeile 12/13). Die Reaktion wird bei einer Temperatur von 10 bis 50 °C oberhalb des Siedepunktes der Säure durchgeführt. Nach der genannten FR '547, (Seite 1, Zeile 24/25 und Seite 2, Zeile 6/7) ist es auch möglich, knapp unter dem theoretischen Siedepunkt der Carbonsäure zu arbeiten, wenn alle Reaktionsteilnehmer sich in der Gasphase befinden. Diese Arbeitstechnik ist durch die Siedepunktsdepression der Carbonsäure infolge der großen Menge Phosgen gegeben.

Weiterhin ist bekannt, die Bildungen von Carbonsäurechloriden aus den Carbonsäuren und Thionylchlorid durch Zugabe von Pyridin zu fördern. Trichloressigsäure erfordert dabei mindestens molare Mengen an Pyridin (J. Chem. Soc., loc. cit. ; Nature Vol. *172*, 29 (1953 ; C.r. Vol. *199*, 1 422 (1934)). Die Verwendung von Pyridin ist kostspielig und aufwendig, da aus Gründen des Umweltschutzes das Pyridin vollständig zurückgeführt werden muß.

Weiterhin ist bekannt, bei der Synthese von Carbonsäurechloriden aus Carbonsäuren und Thionylchlorid N,N-Dimethylformamid und/oder andere Formamide als Katalysator zu verwenden (Schweizer Patent 333 502 ; Helv. Chim. Acta *42*, 1 654 (1959)). Es ist jedoch inzwischen bekannt geworden, daß aus Dimethylformamid und Thionylchlorid das N,N-Dimethylcarbamoylchlorid entsteht (Isw. Akad. Nauk USSR, Chem. Ser. 1971 (3), 513-519 ; zitiert nach C.A. *75*, 48 340m), das bei Mäusen krebserregend wirkt (J. Nat. Cancer Inst. *48* (5), 1 539-1 541 (1972) ; zitiert nach C.A. *77*, 97 540b). Die Verwendung von Dimethylformamid als Katalysator bei der Herstellung von Säurechloriden mit Hilfe von Thionylchlorid ist deshalb nur unter aufwendigen technischen Sicherheitsmaßnahmen vertretbar.

Es wurde nun ein Verfahren zur Herstellung von Carbonsäurehalogeniden gefunden, das dadurch gekennzeichnet ist, daß man eine Carbonsäure der Formel (III)

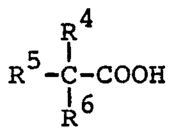

(III)

in der

$R^4$, $R^5$ und $R^6$ unabhängig voneinander Wasserstoff, $C_1$-$C_6$-Alkyl, Vinyl, Phenyl oder Halogen bedeuten, wobei mindestens 1 Substituent Halogen darstellt,

mit einem Säurehalogenid der Formel (II)

$$AOX_2 \qquad (II)$$

in der

A Schwefel oder Kohlenstoff bedeutet und

X für Chlor oder Brom steht,

in Gegenwart von Aktivkohle in flüssiger Phase umsetzt.

Im erfindungsgemäßen Verfahren werden bevorzugt Carbonsäuren der Formel (IV)

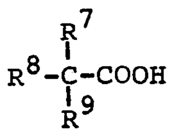

(IV)

eingesetzt, in der

$R^7$, $R^8$ und $R^9$ unabhängig voneinander Wasserstoff, Chlor oder Brom bedeuten, wobei mindestens einer der Substituenten $R^7$ bis $R^9$ Chlor oder Brom bedeutet.

Als Alkyl seien geradkettige oder verzweigte Kohlenwasserstoffreste mit 1 bis 6 Kohlenstoffatomen genannt, wie Methyl, Ethyl, Propyl, Isopropyl, Butyl, Isobutyl, Hexyl.

Als Halogen seien beispielsweise Fluor, Chlor, Brom oder Jod, bevorzugt Chlor oder Brom, genannt.

Als Carbonsäuren für das erfindungsgemäße Verfahren seien beispielsweise genannt : Monochloressigsäure, Dichloressigsäure, Trichloressigsäure, Monobromessigsäure, Dibromessigsäure, Tribromessigsäure, α-Chlorpropionsäure, α-Chlorbuttersäure, α,α-Dichlorpropionsäure, α,α-Dichlorbuttersäure, α-Brompropionsäure, α-Brombuttersäure, α,α-Dibrompropionsäure und α,α-Dibrombuttersäure.

Als Säurehalogenid für das erfindungsgemäße Verfahren sei beispielsweise ein Säurehalogenid der schwefeligen Säure oder der Kohlensäure, bevorzugt der schwefeligen Säure, genannt. Als Säurehalogenide seien beispielsweise die Säurechloride oder die Säurebromide, bevorzugt die Säurechloride, genannt. Säurehalogenide für das erfindungsgemäße Verfahren sind beispielsweise : Thionylchlorid, Thionylbromid, Phosgen, Kohlensäuredibromid. Bevorzugtes Säurehalogenid für das erfindungsgemäße Verfahren ist das Thionylchlorid.

Als Aktivkohle für das erfindungsgemäße Verfahren sei beispielsweise eine Aktivkohle pflanzlicher, tierischer oder mineralischer Herkunft genannt, wie sie in Ullmanns Encyklopädie der technischen Chemie, 4. Auflage, Band 14, Seite 627 beschrieben sind und beispielsweise unter Handelsnamen wie Carboraffin, Eponit oder Supersorbon erhältlich sind. Die Aktivkohle kann als Pulver oder als Granulat im erfindungsgemäßen Verfahren eingesetzt werden. Bevorzugt werden Aktivkohlen eingesetzt, die durch eine Behandlung mit sauer reagierenden Stoffen, wie Zinkchlorid oder Phosphorsäure, aktiviert wurden. Solche aktivierten Aktivkohlesorten tragen vielfach hinter dem Handelsnamen die Bezeichnung Z für Zinkchlorid oder P für Phosphorsäure. Die Aktivkohle wird im erfindungsgemäßen Verfahren in einer Menge von 0,01 bis 10 Gew.-%, bevorzugt 0,05 bis 8 Gew.-% und besonders bevorzugt 0,1 bis 5 Gew.-%, bezogen auf die Menge der eingesetzten Carbonsäure, eingesetzt. Die Aktivkohle verliert ihre Aktivität in Bezug auf das erfindungsgemäße Verfahren bei einem einmaligen Einsatz in der Reaktionsmischung nicht völlig und kann daher besonders wirtschaftlich mehrmals, beispielsweise bis zu 5 mal, eingesetzt werden.

Das erfindungsgemäße Verfahren kann in einem weiten Temperaturbereich durchgeführt werden. Als solcher sei beispielsweise der Bereich von 0 bis 180 °C, bevorzugt von 40 bis 110 °C, genannt. In einer bevorzugten Variante des erfindungsgemäßen Verfahrens wird beim Siedepunkt des am niedrigsten siedenden Anteils des Reaktionsgemisches gearbeitet. Auch bei dieser Variante kann die Reaktionstemperatur durch Druckerhöhung oder Druckerniedrigung innerhalb des genannten Temperaturbereiches variiert werden. Bei Druckerhöhung, beispielsweise unter dem Eigendruck der Reaktionskomponenten, wird in bevorzugter Weise nicht über 150 °C hinausgegangen. In bevorzugter Weise wird bei Normaldruck gearbeitet.

Zur Einstellung einer bestimmten Reaktionstemperatur unter Rückflußbedingungen oder zur Kontrolle der Wärmetönung der Reaktion kann es günstig sein, in Gegenwart eines inerten Verdünnungsmittels zu arbeiten. Als solches seien beispielsweise Kohlenwasserstoffe oder Halogen-Kohlenwasserstoffe genannt, wie Cyclohexan, Ligroin, Benzol, Toluol, Xylol, Chloroform, Tetrachlorkohlenstoff, Perchlorethylen, Tetrachlorethan, Chlorbenzol oder Dichlorbenzol.

Als Molverhältnis zwischen dem eingesetzten Säurehalogenid und der eingesetzten Carbonsäure sei beispielsweise ein Bereich von 0,9 bis 1,5 Mol Säurehalogenid, bevorzugt 1,1 bis 1,2 Mol Säurehalogenid, bezogen auf 1 Mol eingesetzte Carbonsäure, genannt. Die Anwendung des Säurehalogenids in einer Menge von weniger als 1 Mol pro Mol eingesetzte Carbonsäure führt dabei zu unvollständigem Umsatz und damit zu Ausbeuteverlusten. Diese Variante kann jedoch dann günstig sein, wenn der Rückstand des Reaktionsansatzes, der noch unverbrauchte Carbonsäure enthält, in einem folgenden Reaktionsansatz wieder verwendet wird. Die Anwendung eines größeren Überschusses an Säurehalogenid als angegeben führt zu keiner Ausbeuteverbesserung und ist daher wirtschaftlich ungünstig.

In einer besonderen Variante des erfindungsgemäßen Verfahrens wird zunächst überschüssiges Säurehalogenid, bezogen auf die Menge der Carbonsäure, eingesetzt. Nach dem Verbrauch der eingesetzten Carbonsäure wird sodann weitere Carbonsäure in einer solchen Menge zugegeben, daß der

Überschuß des Säurehalogenids ganz oder teilweise aufgebraucht wird. Diese besondere Verfahrensvariante läßt sich günstig bei Verwendung des bevorzugten Thionylchlorids als Säurechlorid durchführen.

Die Reihenfolge des Zusammengebens der Reaktionspartner ist beliebig. Im allgemeinen wird die Aktivkohle vorgelegt und das Säurechlorid und die Carbonsäure gleichzeitig oder nacheinander zugegeben. Höherschmelzende Carbonsäuren können hierbei fest aus einem Feststoffdosiertrichter oder in geschmolzener Form aus einer beheizten Vorlage zugegeben werden. Für den Fall, daß man das Säurechlorid, wie oben beschrieben, in einem größeren Überschuß vorlegt und nach Reaktion der ersten Menge der Carbonsäure weitere Carbonsäure zum Aufbrauchen des Säurechlorides nachdosiert, kann dieses Nachdosieren der Carbonsäure portionsweise oder kontinuierlich erfolgen.

Gegenüber den zum Stande der Technik gehörenden Verfahren zur Herstellung von Carbonsäurechloriden aus den Carbonsäuren und Thionylchlorid wird im erfindungsgemäßen Verfahren durch die Zugabe von Aktivkohle die Reaktionszeit herabgesetzt und die Reaktionstemperatur abgesenkt. Hierdurch werden mögliche Zersetzungsreaktionen und Nebenproduktbildung zurückgedrängt und dabei gleichzeitig die Ausbeute an Säurechlorid erhöht. Die erfindungsgemäße Verwendung von Aktivkohle erlaubt es weiterhin, bei nur geringen molaren Überschüssen an Säurehalogenid, bezogen auf die Carbonsäure, einen hohen Umsatz zu erzielen, während die Verfahren des Standes der Technik hierzu höhere Überschüsse an Thionylchlorid erfordern und dabei ein Thionylchlorid-haltiges Carbonsäurechlorid ergeben. Der niedrige Preis der Aktivkohle gegenüber anderen Zusätzen des Standes der Technik ist wirtschaftlich von besonderem Interesse.

Es ist überraschend, daß die Vorteile des erfindungsgemäßen Verfahrens durch den Zusatz von chemisch inerter Aktivkohle bewirkt werden, während die Verfahren des Standes der Technik, welche Zusätze verwenden, stets solche Zusätze benutzen, die in der Reaktion eingreifen. Das inerte Verhalten der Aktivkohle ist weiterhin aus Gründen der Okologie sehr willkommen.

Im erfindungsgemäßen Verfahren können Carbonsäurehalogenide der Formel (V)

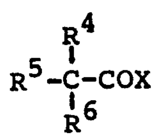

(V)

hergestellt werden, in der

$R^4$, $R^5$, $R^6$ und X die oben gegebene Bedeutung haben.

Die erfindungsgemäß herstellbaren Carbonsäurehalogenide der Formel (V) sind besonders rein und farblos, sie sind frei von gesundheitsschädlichen Nebenprodukten, und sie enthalten wenig oder kein Säurehalogenid der Formel (II).

Carbonsäurehalogenide sind dem Fachmann bekannte Substanzen, die beispielsweise für Acylierungen der verschiedensten Art eingesetzt werden können.

In den folgenden Beispielen werden Aktivkohlen der Handelsbezeichnungen Carboraffin oder Eponit, sowie Knochenkohle eingesetzt. Die Buchstaben Z bzw. P weisen dabei auf eine Aktivierung mit $ZnCl_2$ bzw. $H_3PO_4$ hin.

## Beispiele 1 bis 8

(Beispiele 1 und 3 als Vergleichsbeispiele)

1 Mol Monochloressigsäure (94,5 g) und 1,1 Mol Thionylchlorid (119,0 g) werden mit der in der Tabelle angegebenen Menge von Aktivkohle im Ölbad langsam erhitzt, bis die Ölbadtemperatur 100 °C erreicht ; die Reaktion beginnt bei Raumtemperatur und wird bei erhöhter Temperatur vollständig. Nach Ablauf der angegebenen Reaktionszeit destilliert man das Produkt bis zu einer Innentemperatur von ca. 120 °C ab, wobei $SOCl_2$ mit übergeht, wenn ohne Zusatz von Aktivkohle gearbeitet wird (die Angaben der Katalysatormengen sind Gew.-%, bezogen auf eingesetzte Carbonsäure). Die Werte sind in der folgenden Tabelle zusammengestellt.

| Beispiel | Zusatz | Reakt.zeit h | Ausbeute % | Gehalt des Dest. an Carbonsäurechlorid % |
|---|---|---|---|---|
| 1 | — | 2,5 | 58-60 | ca. 99* |
| 2 | 1 % Carboraffin Z | 2,5 | 84,5 | 99,5 |
| 3 | — | 9 | 75,9 | 99,3 |
| 4 | 1 % Carboraffin Z | 9 | 82,4 | 99,8 |
| 5 | 0,1 % Carboraffin Z | 7,5 | 82,0 | 99,5 |
| 6 | 0,1 % Eponitkohle | 3 | 75,5 | 99,3 |

(Fortsetzung)

| Beispiel | Zusatz | Reakt.zeit h | Ausbeute % | Gehalt des Dest. an Carbonsäurechlorid % |
|---|---|---|---|---|
| 7 | 0,1 % Knochenkohle | 2,5 | 78,4 | 99,1 |
| 8 | 0,1 % Carboraffin P | 2,5 | 78,2 | 99,6 |

* kann noch $SOCl_2$ enthalten

### Beispiel 9 (zum Vergleich)

1 Mol Chloressigsäure und 1,5 Mol Thionylchlorid werden 2,5 h lang bei ca. 80 °C zum Sieden erhitzt. Dann wird alles flüchtige Material abdestilliert und durch Gaschromatographie analysiert.

Man erhält ein Produkt, das 74,3 % Chloracetylchlorid und 20,4 % $SOCl_2$ enthält ; das entspricht einer Ausbeute an Chloracetylchlorid von 94,2 %.

### Beispiel 10

472,5 g (5,0 Mol) Chloressigsäure, 684,0 g (5,75 Mol) Thionylchlorid und 0,5 g Aktivkohle (Carboraffin Z) werden zusammengegeben und unter Rühren langsam bis auf 100 °C Heizbadtemperatur erwärmt.

Nach 2 h werden weitere 3,3 g Chloressigsäure zugegeben, dann wird noch 75 Minuten bei 100 °C Heizbadtemperatur nachgerührt. Durch Destillation erhält man 558,6 g 98,7 %iges Chloracetylchlorid, was einer Ausbeute von 97 % entspricht. Das Produkt enthält laut Gaschromatogramm weniger als 0,5 % Thionylchlorid.

### Beispiel 11

684,0 g Thionylchlorid werden zusammen mit 1,0 g Aktivkohle (Carboraffin Z) zum Sieden erhitzt. Dann gibt man innerhalb von 2,5 Stunden 479,0 g Chloressigsäure geschmolzen aus einer beheizten Vorlage zu, rührt noch 1 h bei ca. 100 °C nach und destilliert dann das erhaltene Chloracetylchlorid. Man erhält eine Ausbeute von 94,5 % ; das Produkt enthält weniger als 0,1 % Thionylchlorid.

### Beispiele 12-15

(Beispiel 12 als Vergleichsbeispiel)

128,9 g (1,0 Mol) Dichloressigsäure, 130,9 g (1,1 Mol) Thionylchlorid und die in der Tabelle angegebenen Mengen an Aktivkohle werden zusammengegeben und unter Rühren langsam bis auf 100 °C Heizbadtemperatur erhitzt. Bei der in der Tabelle angegebenen Temperatur setzt Gasentwicklung ein. Man erwärmt insgesamt während der angegebenen Zeit zum Sieden und destilliert dann ohne weitere Trennmaßnahme ab. Die Werte sind in der folgenden Tabelle zusammengestellt.

| Beispiel | Zusatz | Reakt.-beginn bei °C | Reakt.-zeit h | Nachrühr-zeit h | Ausbeute % | Gehalt % | Gehalt $SOCl_2$ % |
|---|---|---|---|---|---|---|---|
| 12 | — | > 70 | 3 | 2,5 | 73,4 | 70,1 | 23,7 |
| 13 | 1 % Carboraffin Z | 50 | 0,5 | 5,0 | 88,8 | 90,0 | — |
| 14 | 1 % Carboraffin P | 50 | 2,0 | 1,0 | 73,0 | 99,4 | < 0,1 |
| 15 | 1 % Eponitkohle | 50 | 2,0 | 1,0 | 77,6 | 99,6 | < 0,1 |

### Beispiel 16

163,4 g (1 Mol) Trichloressigsäure, 130,9 g (1,1 Mol) Thionylchlorid und 8,1 g Aktivkohle (Carboraffin Z) werden 6 h lang zum Sieden erhitzt. Dann wird ohne weitere Reinigungsoperation abdestilliert. Man erhält 139,3 g Produkt, das laut gaschromatographischer Analyse 98,0 % Trichloracetylchlorid enthält (= 75,1 % Ausbeute).

Beispiel 17

In einem Versuch, der wie Beispiel 16, jedoch ohne Zusatz von Aktivkohle durchgeführt wurde, wurde kein Trichloracetylchlorid gebildet.

**Ansprüche**

1. Verfahren zur Herstellung von Carbonsäurehalogeniden, dadurch gekennzeichnet, daß man eine Carbonsäure der Formel

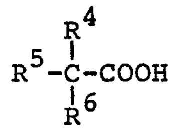

in der
$R^4$, $R^5$ und $R^6$ unabhängig voneinander Wasserstoff, $C_1$-$C_6$-Alkyl, Vinyl, Phenyl oder Halogen bedeuten, wobei mindestens 1 Substituent Halogen darstellt,
mit einem Säurehalogenid der Formel

$$AOX_2$$

in der
A Schwefel oder Kohlenstoff bedeutet und
X für Chlor oder Brom steht,
in Gegenwart von Aktivkohle in flüssiger Phase umsetzt.

2. Verfahren nach Anspruch 1, dadurch gekennzeichnet, daß man als Säurehalogenid Thionylchlorid in einer Menge von 0,9 bis 1,5 Mol pro Mol Carbonsäure einsetzt.

3. Verfahren nach Ansprüchen 1 und 2, dadurch gekennzeichnet, daß man 0,01 bis 10 Gew.-% Aktivkohle, bezogen auf die eingesetzte Carbonsäure, anwendet.

4. Verfahren nach Ansprüchen 1 bis 3, dadurch gekennzeichnet, daß man eine durch saure Stoffe aktivierte Aktivkohle einsetzt.

5. Verfahren nach Ansprüchen 1 bis 4, dadurch gekennzeichnet, daß man das Säurehalogenid zunächst im Überschuß gegenüber der Carbonsäure einsetzt und dann nach dem Verbrauch der Carbonsäure noch soviel weitere Carbonsäure portionsweise oder kontinuierlich nachgibt, daß der Überschuß an Säurehalogenid ganz oder teilweise aufgebraucht wird.

6. Verfahren nach Anspruch 1 bis 5, dadurch gekennzeichnet, daß man Carbonsäuren der Formel

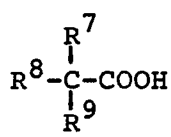

einsetzt, in der
$R^7$, $R^8$ und $R^9$ unabhängig voneinander Wasserstoff, Chlor oder Brom bedeuten, wobei mindestens 1 Substituent Chlor oder Brom bedeutet.

7. Verfahren nach Anspruch 1 bis 6, dadurch gekennzeichnet, daß man bei 0 bis 180 °C arbeitet.

8. Verfahren nach Anspruch 1 bis 6, dadurch gekennzeichnet, daß man bei 40 bis 110 °C arbeitet.

9. Verfahren nach Anspruch 1 bis 8, dadurch gekennzeichnet, daß man in Gegenwart eines inerten Verdünnungsmittels arbeitet.

10. Verfahren nach Anspruch 1 bis 9, dadurch gekennzeichnet, daß man bei der Rückflußtemperatur der am niedrigsten siedenden Komponente des Reaktionsgemisches arbeitet.

**Claims**

1. Process for the preparation of carboxylic acid halides, characterised in that a carboxylic acid of the formula

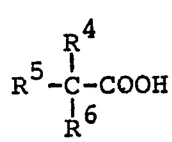

in which

$R^4$, $R^5$ and $R^6$ independently of one another denote hydrogen, $C_1$-$C_6$-alkyl, vinyl, phenyl or halogen, at least 1 substituent being halogen,

is reacted with an acid halide of the formula

$$AOX_2$$

in which

A denotes sulphur or carbon and

X represents chlorine or bromine,

in the presence of active charcoal in the liquid phase.

2. Process according to Claim 1, characterised in that the acid halide employed is thionyl chloride, in an amount of 0.9 to 1.5 mols per mol of carboxylic acid.

3. Process according to Claims 1 and 2, characterised in that 0.01 to 10 % by weight of active charcoal, relative to the carboxylic acid employed, is used.

4. Process according to Claims 1 to 3, characterised in that an active charcoal activated by acid substances is employed.

5. Process according to Claims 1 to 4, characterised in that the acid halide is initially employed in excess, compared with the carboxylic acid, and, after the carboxylic acid has been consumed further carboxylic acid is then subsequently added, in portions or continuously, in an amount such that all or some of the excess acid halide is used up.

6. Process according to Claims 1 to 5, characterised in that carboxylic acids of the formula

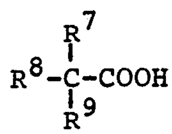

are employed, in which

$R^7$, $R^8$ and $R^9$ independently of one another denote hydrogen, chlorine or bromine, at least 1 substituent being chlorine or bromine.

7. Process according to Claims 1 to 6, characterised in that it is carried out at 0 to 180 °C.

8. Process according to Claims 1 to 6, characterised in that it is carried out at 40 to 110 °C.

9. Process according to Claims 1 to 8, characterised in that it is carried out in the presence of an inert diluent.

10. Process according to Claims 1 to 9, characterised in that it is carried out at the reflux temperature of the constituent of the reaction mixture which has the lowest boiling point.

**Revendications**

1. Procédé pour la préparation d'halogénures d'acides carboxyliques, caractérisé en ce que l'on fait réagir en phase liquide un acide carboxylique de formule

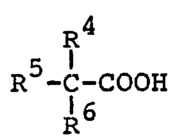

dans laquelle

$R^4$, $R^5$ et $R^6$ représentent chacun, indépendamment les uns des autres, l'hydrogène, un groupe alkyle en $C_1$-$C_6$, vinyle, phényle ou un halogène, l'un au moins des substituants étant un halogène, avec un halogénure d'acide de formule

$$AOX_2$$

dans laquelle

A représente le soufre ou le carbone et

X représente le chlore ou le brome,

en présence de charbon actif.

2. Procédé selon la revendication 1, caractérisé en ce que l'on utilise en tant qu'halogénure d'acide le chlorure de thionyle en quantité de 0,9 à 1,5 mole par mole d'acide carboxylique.

3. Procédé selon les revendications 1 et 2, caractérisé en ce que l'on utilise de 0,01 à 10 % en poids

de charbon actif par rapport à l'acide carboxylique mis en œuvre.

4. Procédé selon les revendications 1 à 3, caractérisé en ce que l'on utilise un charbon actif activé par des substances acides.

5. Procédé selon les revendications 1 à 4, caractérisé en ce que l'on met d'abord l'halogénure d'acide en œuvre en excès par rapport à l'acide carboxylique puis, après consommation de l'acide carboxylique, on ajoute encore, par portions ou en continu, des compléments d'acide carboxylique en quantité telle que l'excès d'halogénure d'acide soit consommé en totalité ou en partie.

6. Procédé selon les revendications 1 à 5, caractérisé en ce que l'on met en œuvre des acides carboxyliques de formule

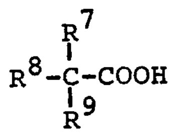

dans laquelle

$R^7$, $R^8$ et $R^9$ représentent chacun, indépendamment les uns des autres, l'hydrogène, le chlore ou le brome, l'un au moins des substituants étant le chlore ou le brome.

7. Procédé selon les revendications 1 à 6, caractérisé en ce que l'on opère à une température de 0 à 180 °C.

8. Procédé selon les revendications 1 à 6, caractérisé en ce que l'on opère à une température de 40 à 110 °C.

9. Procédé selon les revendications 1 à 8, caractérisé en ce que l'on opère en présence d'un diluant inerte.

10. Procédé selon les revendications 1 à 9, caractérisé en ce que l'on opère à la température de reflux du composant du mélange de réaction qui bout le plus bas.